# EUROPEAN PATENT APPLICATION

(11) **EP 4 434 432 A1**
(43) Date of publication of application: **25.09.2024**
(21) Application number: 21964730.2
(22) Date of filing: 18.11.2021
(51) Int. Cl.: A61B 1/045

(54) **IMAGE DISPLAY DEVICE, IMAGE DISPLAY METHOD, AND RECORDING MEDIUM**

(71) Applicant: NEC Corporation, 108-8001 Tokyo (JP)
(72) Inventor: KAMIJO, Kenichi, Tokyo 108-8001 (JP); TAKAHASHI, Ikuma, Tokyo 108-8001 (JP); OKUTSU, Motoyasu, Tokyo 108-8001 (JP); OHTSUKA, Shota, Tokyo 108-8001 (JP); KIMURA, Tatsu, Tokyo 108-8001 (JP)
(74) Representative: Betten & Resch
(86) International application number: PCT/JP2021/042364
(87) International publication number: WO 2023/089715

(57) **Abstract**

In an image display device, a storage means stores past examination data in which identification information of a subject is associated with a lesion image obtained by imaging a lesion, positional information of the lesion and lesion information including treatment performed for the lesion. An identification information acquisition means acquires identification information of a subject. The lesion information acquisition means acquires the lesion information corresponding to the acquired identification information from the storage means. Then, the display control means display the acquired lesion information on the display device.

## Description

### TECHNICAL FIELD

The present disclosure relates to the display of endoscopic examination images.

### BACKGROUND ART

Conventionally, when an endoscopic examination is performed, the amount of information of the lesion positions found in the previous examination depends on the amount of information recorded in the electronic medical record, and it is difficult to efficiently manage and refer to the information obtained in the past examinations. From this point of view, Patent Document 1 proposes a method for synchronously displaying a past captured image on the display unit together with the current captured image in the endoscope system.

### PRECEDING TECHNICAL REFERENCES

### PATENT DOCUMENT

Patent Document 1: Japanese Patent Application Laid-open under No. 2012-170774

### SUMMARY

### PROBLEM TO BE SOLVED

However, even by Patent Document 1, it is not always possible to refer to sufficient information of the lesions found in the past endoscopic examination in a suitable manner.

It is an object of the present disclosure to provide an image display device that enables the current examination while considering the information about the treatment of the lesions found in the past endoscopic examinations.

### MEANS FOR SOLVING THE PROBLEM

According to an example aspect of the present disclosure, there is provided an image display device comprising:
a storage means configured to store past examination data in which identification information of a subject is associated with a lesion image obtained by imaging a lesion, positional information of the lesion and lesion information including treatment performed for the lesion;
an identification information acquisition means configured to acquire identification information of a subject;
a lesion information acquisition means configured to acquire the lesion information corresponding to the acquired identification information from the storage means; and
a display control means configured to display the lesion information on a display device.

According to another example aspect of the present disclosure, there is provided an image display method comprising:
acquiring identification information of a subject;
acquiring lesion information corresponding to the acquired identification information from a storage, the storage storing past examination data in which identification information of a subject is associated with a lesion image obtained by imaging a lesion, positional information of the lesion and lesion information including treatment performed for the lesion; and
displaying the lesion information on a display device.

According to still another example aspect of the present disclosure, there is provided a recording medium recording a program, the program causing a computer to execute processing of:
acquiring identification information of a subject;
acquiring lesion information corresponding to the acquired identification information from a storage, the storage storing past examination data in which identification information of a subject is associated with a lesion image obtained by imaging a lesion, positional information of the lesion and lesion information including treatment performed for the lesion; and
displaying the lesion information on a display device.

### EFFECT

According to the present disclosure, during the endoscopic examination, it is possible to proceed with the examination while considering information about the treatment of the lesion found in the past endoscopic examinations.

### BRIEF DESCRIPTION OF THE DRAWINGS

[FIG. 1] FIG. 1 is a block diagram showing a schematic configuration of an endoscopic examination system.
[FIG. 2] FIG. 2 is a block diagram showing a hardware configuration of an image processing device.
[FIG. 3] FIG. 3 is a block diagram showing a functional configuration of the image processing device.
[FIG. 4] FIG. 4 schematically shows a structure of a large intestine.
[FIG. 5] FIG. 5 shows an example of past data stored in a database.
[FIG. 6] FIG. 6 shows a display example by a past data display mode.
[FIG. 7] FIG. 7 shows another display example by the past data display mode.
[FIG. 8] FIG. 8 shows still another display example by the past data display mode.
[FIG. 9] FIG. 9 shows a display example by a parallel display mode.
[FIG. 10] FIG. 10 shows a display example by a display mode for patient.
[FIG. 11] FIG. 11 is a flowchart of image display processing by the image processing device.
[FIG. 12] FIG. 12 is a block diagram showing a functional configuration of an image display device according to a second example embodiment.
[FIG. 13] FIG. 13 is a flowchart of processing executed by the image display device according to the second example embodiment.

### EXAMPLE EMBODIMENTS

Preferred example embodiments of the present disclosure will be described with reference to the accompanying drawings.

### <First example embodiment>

### [System Configuration]

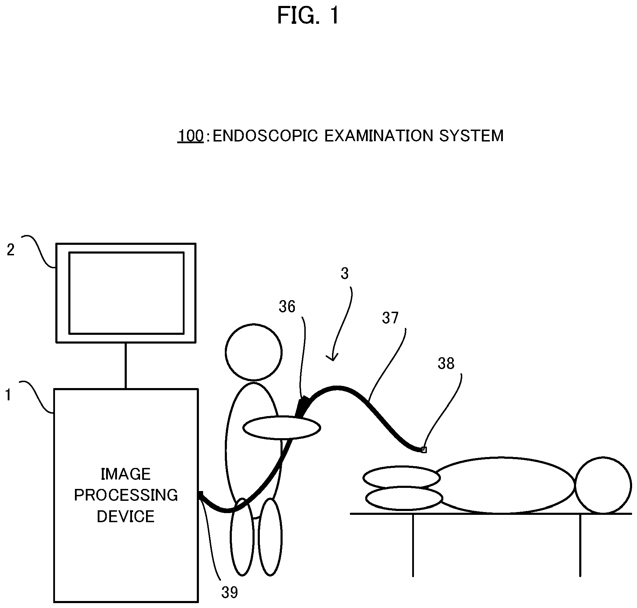

FIG. 1 shows a schematic configuration of an endoscopic examination system 100. At the time of the examination (including treatments) using the endoscope, the endoscopic examination system 100 acquires and displays the lesion information including the lesion images obtained by the past examinations and the information related to the lesion (hereinafter, referred to as "related information"). In particular, the endoscopic examination system 100 of this example embodiment is characteristic in displaying the related information including information about the treatment performed on the lesions detected in the past examinations. This makes it possible to carry out the examination while accurately observing and judging the subsequent progress of the lesion, in consideration of the treatment performed for the lesion in the past examination.

As shown in FIG. 1, the endoscopic examination system 100 mainly includes an image processing device 1, a display device 2, and an endoscope 3 connected to the image processing device 1.

The image processing device 1 acquires a moving image (hereinafter also referred to as an "endoscopic image Ic") captured by the endoscope 3 during the endoscopic examination from the endoscope 3 and displays display data for the check by the examiner of the endoscopic examination on the display device 2. Specifically, the image processing device 1 acquires a moving image of organs captured by the endoscope 3 as the endoscopic image Ic during the endoscopic examination. In addition, when the examiner finds a lesion during the endoscopic examination, he or she operates the endoscope 3 to input an imaging instruction of the lesion position. Based on the imaging instruction by the examiner, the image processing device 1 generates a lesion image capturing the lesion position. Specifically, the image processing device 1 generates the lesion image which is a still image, from the endoscopic image Ic which is a moving image, on the basis of the imaging instruction of the examiner.

The display device 2 is a display or the like for displaying images on the basis of the display signal supplied from the image processing device 1.

The endoscope 3 mainly includes an operation unit 36 used by the examiner to input instructions such as air supply, water supply, angle adjustment, and the imaging instruction, a shaft 37 having flexibility and inserted into an organ of a subject to be examined, a tip portion 38 with a built-in imaging unit such as an ultra-compact imaging element, and a connection unit 39 for connection with the image processing device 1.

While the following explanation is mainly given on the processing of endoscopic examination for a large intestine, the subject of examination may be gastrointestinal (digestive organs) such as the stomach, esophagus, small intestine, and duodenum, as well as the large intestine.

In addition, the part to be detected in the endoscopic examination is not limited to the lesion part, but may be any part (also referred to as a "region of interest") in which the examiner needs attention. The region of interest may be, for example, a lesion part, a part where inflammation has occurred, a part where a surgical scar or other cut has occurred, a part where a fold or protrusion has occurred, or a part where the tip portion 38 of the endoscope 3 is likely to contact (easily stuck) the intraluminal wall surface.

### [Hardware configuration]

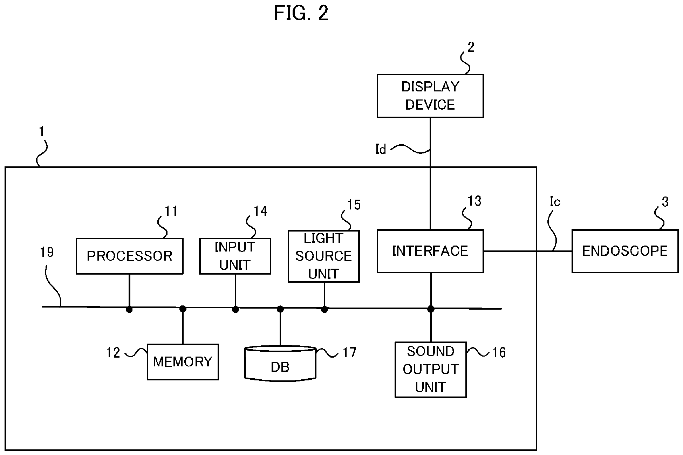

FIG. 2 shows a hardware configuration of the image processing device 1. The image processing device 1 mainly includes a processor 11, a memory 12, an interface 13, an input unit 14, a light source unit 15, a sound output unit 16, and a data base (hereinafter referred to as "DB") 17. These elements are connected with each other via a data bus 19.

The processor 11 executes a predetermined processing by executing a program stored in the memory 12. The processor 11 is a processor such as a CPU (Central Processing unit), a GPU (Graphics Processing unit), and a TPU (Tensor Processing unit). The processor 11 may be configured by multiple processors. The processor 11 is an example of a computer.

The memory 12 is configured by various volatile memories used as a working memory and non-volatile memories for storing information needed for the processing of the image processing device 1, such as a RAM (Random Access Memory) and a ROM (Read Only Memory). Incidentally, the memory 12 may include an external storage device such as a hard disk connected to or incorporated in the image processing device 1, and may include a storage medium such as a removable flash memory or a disk medium. The memory 12 stores a program for the image processing device 1 to execute processing in the present example embodiment.

Also, the memory 12 temporarily stores a series of endoscopic images Ic taken by the endoscope 3 in the endoscopic examination, based on the control of the processor 11. Further, the memory 12 temporarily stores the lesion images taken in response to the imaging instructions by the examiner during the endoscopic examination. These images are stored in the memory 12 in association with, for example, subject identification information (e.g., the patient ID) and time stamp information, etc.

The interface 13 performs an interface operation between the image processing device 1 and the external devices. For example, the interface 13 supplies the display data Id generated by the processor 11 to the display device 2. Also, the interface 13 supplies the illumination light generated by the light source unit 15 to the endoscope 3. Also, the interface 13 supplies an electrical signal indicating the endoscopic image Ic supplied from the endoscope 3 to the processor 11. The interface 13 may be a communication interface such as a network adapter for wired or wireless communication with an external device, or may be a hardware interface compliant with a USB (Universal Serial Bus), SATA (Serial Advanced Technology Attachment), etc.

The input unit 14 generates an input signal based on the operation of the examiner. The input unit 14 is, for example, a button, a touch panel, a remote controller, a voice input device, or the like. The light source unit 15 generates the light to be delivered to the tip portion 38 of the endoscope 3. The light source unit 15 may also incorporate a pump or the like for delivering water or air to be supplied to the endoscope 3. The sound output unit 16 outputs the sound based on the control of the processor 11.

The DB 17 stores the endoscopic images acquired by past endoscopic examinations of the subject, and the lesion information. The lesion information includes the lesion images and the related information. The DB 17 may include an external storage device, such as a hard disk connected to or incorporated in the image processing device 1, and may include a storage medium, such as a removable flash memory. Instead of providing the DB 17 in the endoscopic examination system 100, the DB 17 may be provided in an external server or the like to acquire associated information from the server through communication.

### [Functional configuration]

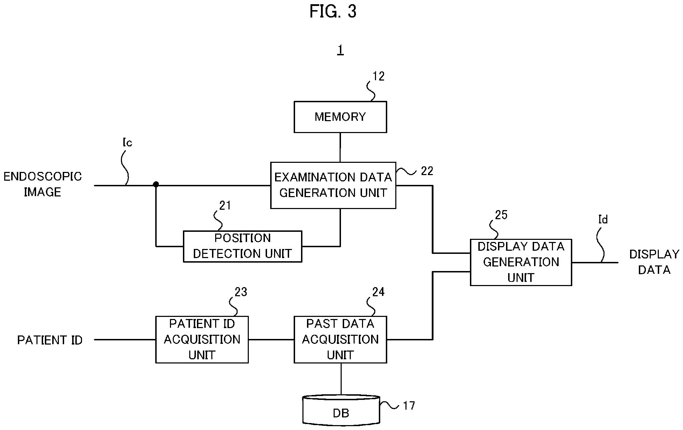

FIG. 3 is a block diagram showing a functional configuration of the image processing device 1. The image processing device 1 functionally includes a position detection unit 21, an examination data generation unit 22, a patient ID acquisition unit 23, a past data acquisition unit 24, and a display data generation unit 25.

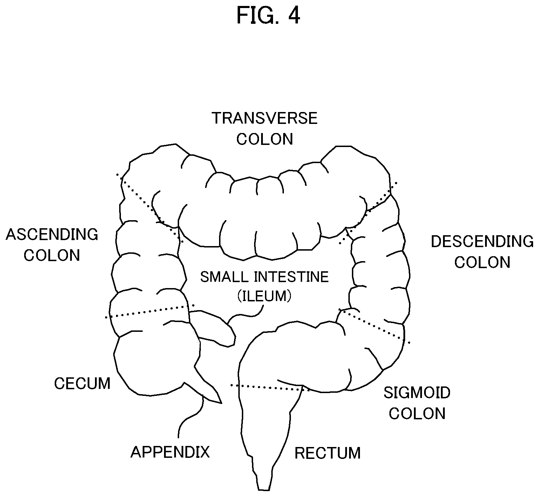

The image processing device 1 receives the endoscopic image Ic from the endoscope 3. The endoscopic image Ic is inputted into the position detection unit 21 and the examination data generation unit 22. The position detection unit 21 detects the position of the endoscope 3, i.e., the imaging position of the endoscopic image, based on the endoscopic image Ic. Specifically, the position detection unit 21 detects the imaging position by image analysis of the inputted endoscopic image Ic. Here, the imaging position may be three-dimensional coordinates in the organ of the examination target, but may be at least an information indicating one of a plurality of parts of the organ of the examination target. For example, as shown in FIG. 4, the large intestine includes multiple parts such as the cecum, the ascending colon, the transverse colon, the descending colon, the sigmoid colon, and the rectum. Therefore, when the examination target is the large intestine, the position detection unit 21 may detect at least which one of the above-described parts the imaging position belongs to.

Specifically, the position detection unit 21 can estimate which part of the large intestine the imaging position at that time belongs to, based on the pattern of the mucous membrane, the presence or absence of the folds, the shape of the folds in the endoscopic image, the number of the folds passed by the movement of the endoscope 3, and the like. The position detection unit 21 may estimate the imaging position by estimating the movement speed of the endoscope 3 based on the endoscopic image and calculating the movement distance in the large intestine based on the movement speed and the time. In addition to the image analysis of the endoscopic image, the position detection unit 21 may detect the imaging position using the insertion length of the endoscope 3 inserted into the organ. In the present example embodiment, the detection method of the imaging position in the organ of the examination target is not limited to a specific method. The position detection unit 21 outputs the detected imaging position to the examination data generation unit 23.

The examination data generation unit 22 generates the examination data at the imaging position based on the endoscopic image Ic and the imaging position detected by the position detection unit 21, and temporarily stores the examination data in the memory 12. Specifically, when the examiner operates the endoscope 3, the examination data generation unit 22 generates the examination data including the endoscopic image Ic captured by the endoscope 3 and the imaging information including the patient name, the patient ID, the examination date and time, the imaging position, and the like, and stores the examination data in the memory 12. Also, the examination data generation unit 22 outputs the generated examination data to the display data generation unit 25.

Further, the patient ID of the patient is inputted to the image processing device 1 through the input unit 14. The patient ID is information that uniquely identifies the patient, and may be a personal identification number such as a My Number as well as an ID uniquely assigned to each patient. The patient ID acquisition unit 23 acquires the patient ID inputted through the input unit 14 and outputs the patient ID to the past data acquisition unit 24.

The past data acquisition unit 24 acquires the past data corresponding to the patient ID inputted from the patient ID acquisition unit 23 from the DB 17. The past data include the lesion information obtained during the past endoscopic examinations of the patient. As described above, the DB 17 stores the past data obtained by the past endoscopic examinations for a large number of patients. The past data acquisition unit 24 acquires the past data from the DB 17 based on the patient ID of the subject, and outputs the past data to the display data generation unit 25.

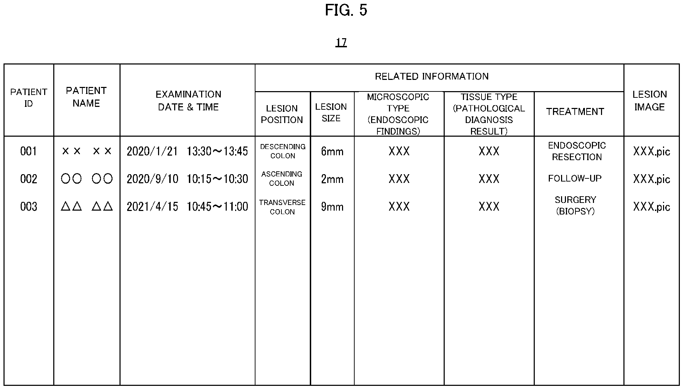

FIG. 5 shows an example of the past data stored in the DB 17. As shown, the DB 17 stores the patient name, the examination date and time, the related information, and the lesion image in association with the patient ID. The "patient name" is the name of the patient who is the subject, and the "examination date and time" is the date and time of the endoscopic examination performed in the past for that patient.

The "related information" includes the lesion position, the lesion size, the macroscopic type (endoscopic findings), the tissue type (pathological diagnosis result), and the treatment. The "lesion position" is the position where the lesion was found, which is concretely one of the plurality of parts that make up the large intestine. The "lesion size" is the size of the lesion found. The "macroscopic type" is the classification result of the lesion by the examiner's naked eye, which is the so-called endoscopic findings. The "tissue type" is the pathologic diagnosis result of the tissue at the part of the lesion. The "treatment" is the treatment which was done for the lesion found in the endoscopic examination. As the example of the treatment, the "endoscopic resection" indicates that the lesion part was removed by the endoscope, the "follow-up" indicates that the lesion part was left because the lesion size was smaller than a predetermined value, and the "surgery (biopsy)" indicates that the tissue of the lesion part was taken for the purpose of the pathological diagnosis. The "lesion image" is an image obtained by imaging the lesion part, which is shown in FIG. 5 by the file name of the image.

Incidentally, the example of FIG. 5 shows the case in which one lesion is found for each patient, for convenience. However, when multiple lesions are found for one patient, multiple related information and multiple images are stored in the DB 17 for each patient as the past data.

The display data generation unit 25 generates the display data Id using the examination data inputted from the examination data generation unit 22 and the past data inputted from the past data acquisition unit 24, and outputs the display data Id to the display device 2. The display data generation unit 25 has a past data display mode and a parallel display mode as main display modes. Here, the "past data display mode" is a mode for displaying only the past data. The past data display mode is used, for example, when the examiner pre-checks the past data of a patient before starting the endoscopic examination of the patient. The "parallel display mode" is a mode in which the current examination data and the past data are displayed in parallel. The parallel display mode is used, for example, to compare and observe the lesion currently found and found in the past during endoscopic examination. In either mode, the examiner can display and confirm the past data of the patient subjected to the endoscopic examination.

### [Display example]

Next, display examples by the display device 2 will be described.

### (Past data display mode)

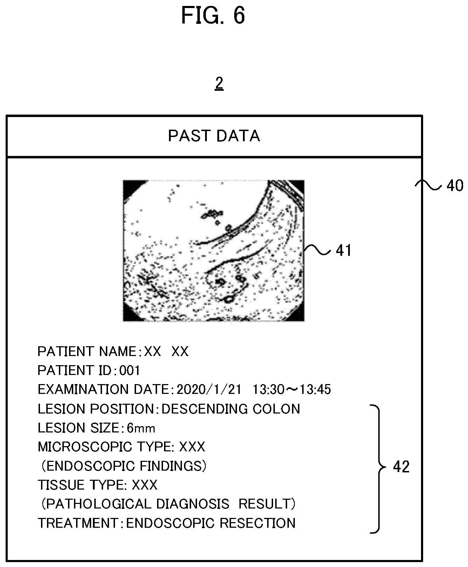

FIG. 6 shows a display example by the past data display mode. In this example, one lesion is included in the past data of a patient, and the lesion image and the related information of the one lesion are displayed.

Specifically, in the display area 40 in the display example of FIG. 6, the lesion image 41 and the related information 42 are displayed in addition to the basic information such as the patient name, the patient ID and the examination date and time. The lesion image 41 is a photographed image of the lesion part found in the past endoscopic examination. The related information 42 is information about the lesion included in lesion image 41 and includes the lesion position, the lesion size, the macroscopic type (endoscopic findings), the tissue type (pathological diagnosis result), and the treatment. Thus, since the lesion position, the lesion size, the macroscopic type, the tissue type, or the like are displayed as the related information in the present example embodiment, the examiner can easily grasp the features of the lesion by viewing the related information 42 together with the lesion image 41. Also, since the examiner can obtain information of the treatment as the related information, he or she can know what kind of treatment was made on the lesion in the past examination. Therefore, the examiner can appropriately judge the progress of the lesion after the previous treatment and the treatment to be made in the future by observing the present lesion image in consideration of the previous treatment.

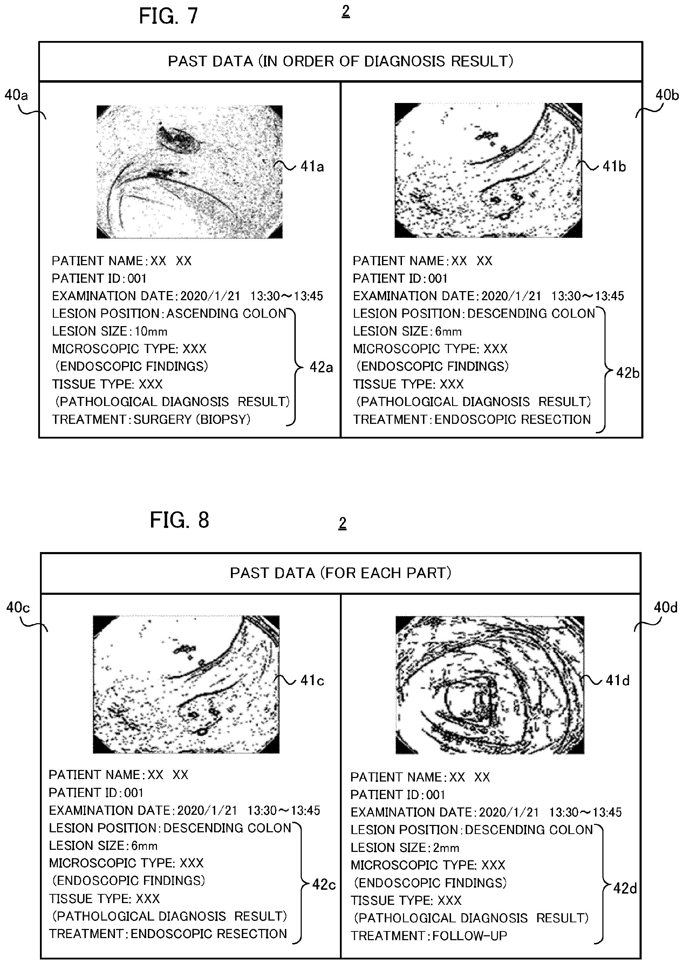

FIG. 7 shows another display example by the past data display mode. This example is for the case where the past data includes a plurality of lesions. It is now assumed that the patient's past data includes three lesions, wherein the treatment for the first lesion was the surgery (biopsy), the treatment for the second lesion was the endoscopic resection, and the treatment for the third lesion was the follow-up. In this case, the image processing device 1 displays, among the plurality of lesions, the lesion whose diagnostic result is serious with higher priority. In the above-described example, the image processing device 1 displays, among the plurality of lesions included in the past data, the first lesion whose diagnosis result is the most serious and the second lesion which is the next serious. Specifically, the display area 40a on the left side of FIG. 7 displays the lesion image 41a of the first lesion and the related informational 42a. In addition, the display area 40b on the right side of FIG. 7 displays the lesion image 41b of the second lesion and the related informational 42b. Thus, when the past data of a certain patient includes a plurality of lesions, by displaying the lesion whose diagnosis result is serious with a higher priority, the examiner can carry out the examination in consideration of the seriousness of the lesions.

FIG. 8 shows another display example in the past data display mode. This example is also an example when the past data includes a plurality of lesions. In this example, when the past data of a certain patient includes a plurality of lesions, the past data are collectively displayed for each part where the lesions are found. In the example of FIG. 8, multiple lesions are found in the descending colon, and the image processing device 1 simultaneously displays two lesions found in the descending colon. Specifically, the display area 40c on the left side of FIG. 8 displays the lesion image 41c of one lesion found in the descending colon and the related information 42c. Also, the display area 40d on the right side of FIG. 8 displays the lesion image 41d of another lesion found in the descending colon and the related information 42d.

When the lesion is found in a plurality of parts, a selection screen of the part to be displayed may be displayed on the display device 2, so that the examiner can select the part to be displayed. For example, if the lesions are found in the descending colon and the transverse colon, a selection button to select one of the the descending colon and the transverse colon may be displayed on display 2 to allow the examiner to select one of them. In this case, the image processing device 1 displays the lesion image and the related information found at the part that the examiner selected on the selection screen. This allows the examiner to collectively display and view the lesions found at the part by selecting the part to be viewed.

In addition, priorities may be given in advance for the parts to be examined, and when the lesion is found in a plurality of parts, the part to be displayed may be determined according to the priorities. For example, if one of the plurality of parts in which the lesion tends to worsen or the lesion tends to be overlooked is known to some extent, such part may be preferentially displayed.

In the example of FIG. 8, two lesions are displayed. However, when three or more lesions are found for one part, three or more lesions may be displayed simultaneously depending on the display space of the display device 2. In addition, when a plurality of lesions is found in the same part and the lesion images and the related information of all lesions cannot be displayed, the the lesions whose diagnostic result is serious may be displayed with high priority as in the example of FIG. 7.

### (Parallel display mode)

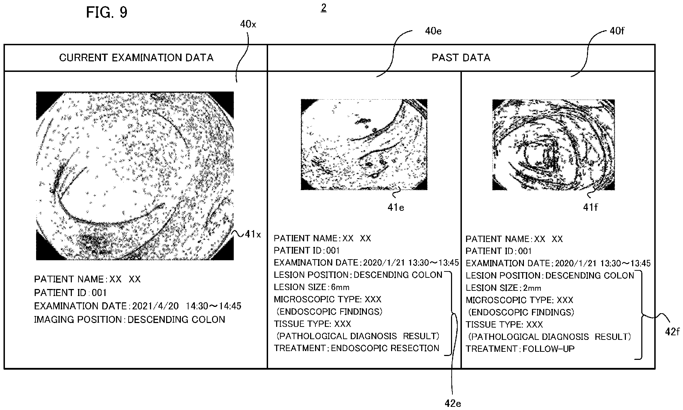

FIG. 9 shows a display example by the parallel display mode. The parallel display mode is a mode in which the current examination data and the past data are displayed in parallel during the endoscopic examination. As shown in FIG. 9, the left display area 40x displays the current endoscopic image 41x and the imaging data such as the patient name, the patient ID, the examination date and time, and the imaging position.

On the other hand, the display areas 40e and 40f on the right show the past data. Specifically, the display area 40e displays the lesion image 41e and the related information 42e as one the lesion information included in the past data. In addition, the display area 40f displays the lesion image 41f and the related information 42f as another lesion information included in the past data.

At this time, the image processing unit 1 displays the lesion information of the past data found at the same imaging position as the imaging position of the endoscopic image 41x displayed as the current examination data. In the example of FIG. 9, since the endoscopic image 41x displayed on the display area 40x is the image of the descending colon, the image processing unit 1 acquires the the lesion information found in the descending colon from the past data of this patient, and displays it as the past data on the display areas 40e and 40f. By this, the current endoscopic image and the past lesion information of the same position can be displayed side by side. Further, when the examination progresses from the state of FIG. 9 and the imaging position moves from the descending colon to the sigmoid colon, the image processing unit 1 displays the endoscopic image 41x of the sigmoid colon. Also, the image processing unit 1 acquires the lesion information found in the sigmoid colon from the past data and displays it on the display areas 40e and/or 40f. That is, the image processing device 1 automatically changes the position of the lesion information of the past data to be displayed, in accordance with the current examination position. This allows the examiner to always refer to the past data corresponding to the imaging position of the current endoscopic image.

### (Modification)

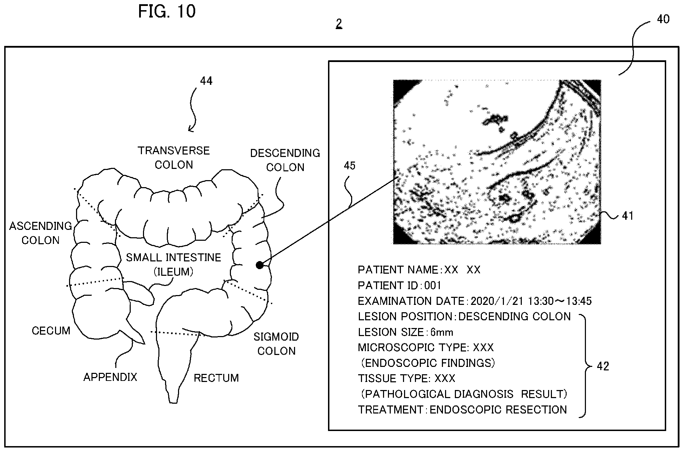

In addition to the above past data display mode and parallel display mode, a display mode for patient may be provided. The "display mode for patient " is a mode mainly used when the examiner explains the endoscopic examination result to the patient. FIG. 10 shows a display example of the display mode for patient. In the display mode for patient, a schematic view of an organ (in the present example embodiment, the large intestine) to be examined is displayed in addition to the lesion information. In FIG. 10, the lesion information displayed on the display area 40 is based on the past data and is the same as the lesion information shown in FIG. 6. In addition, in FIG. 10, a schematic diagram 44 schematically illustrating the shape of the large intestine is displayed. Further, the part where the lesion displayed on the display area 40 was found (the descending colon in this example) is indicated by the line 45. Thus, displaying the lesion information together with the schematic diagram of the organ enables an easy-to-understand explanation for the patient with no detailed knowledge of the organ.

### [Image Display Processing]

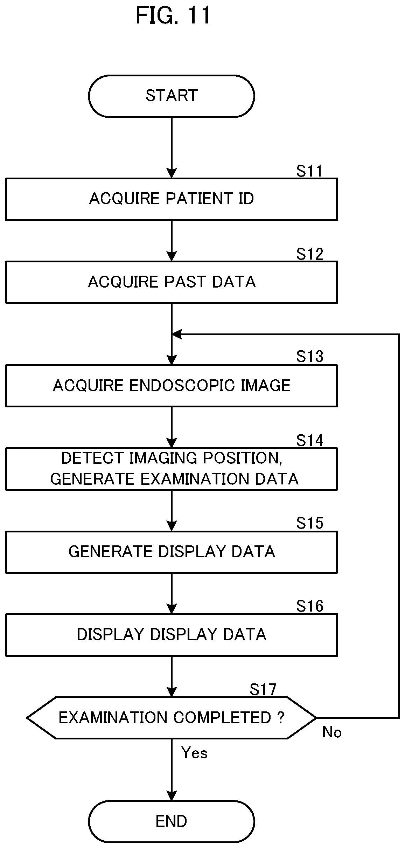

Next, image display processing for performing the above-described display will be described. FIG, 11 is a flowchart of the image display processing by the image processing device 1. This processing is realized by the processor 11 shown in FIG. 2, which executes a pre-prepared program and operates as each element shown in FIG. 3. Incidentally, FIG. 11 is an example of the parallel display mode described above.

First, the patient ID acquisition unit 23 acquires the patient ID of the patient to be examined (step S 11). Next, the past data acquisition unit 24 acquires the past data corresponding to the acquired patient ID from the DB 17 (step S12).

Next, when the examination starts, the position detection unit 21 and the examination data generation unit 22 acquire the endoscopic image Ic from the endoscope 3 (step S13). Next, the position detection unit 21 detects the imaging position based on the endoscopic image Ic or the like, and the examination data generation unit 22 generates the examination data including the endoscopic image and the imaging information at the imaging position as shown in the display area 40x of FIG. 9 (step S14).

Next, the display data generation unit 25 uses the current examination data generated by the examination data generation unit 22 and the past data acquired by the past data acquisition unit 24 to generate the display data as illustrated in FIG. 9, and outputs the display data to the display device 2 (step S15). The display device 2 displays the received display data (step S16). Thus, the display like the display example of FIG. 9 is performed.

Next, the image processing device 1 determines whether or not the examination is completed (step S17). For example, the image processing device 1 determines that the examination is completed when the examiner makes an operation of ending the examination to the image processing device 1 or the endoscope 3. Further, the image processing device 1 may automatically determine that the examination is completed when the captured image becomes an image outside the organ by the image analysis of the captured image by the endoscope 3. If it is determined that the examination is not been completed (step S17: No), the processing returns to step S13. On the other hand, if it is determined that the examination is completed (step S17: Yes), the image display processing ends.

### <Second example embodiment>

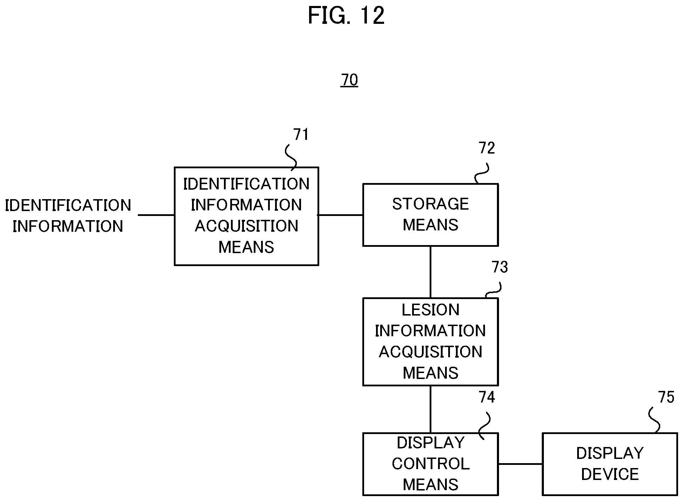

FIG. 12 is a block diagram showing a functional configuration of an image display device according to a second example embodiment. The image display device 70 includes an identification information acquisition means 71, a storage means 72, a lesion information acquisition means 73, a display control means 74 and a display device 75.

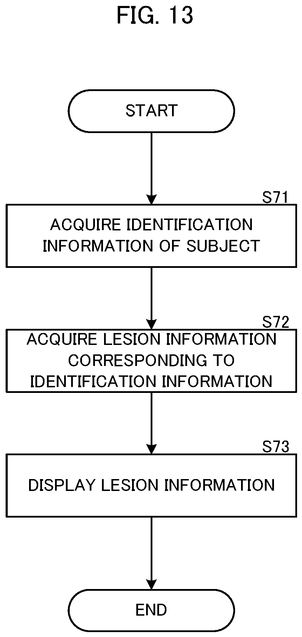

FIG. 13 is a flowchart of processing by the image display device according to the second example embodiment. The identification information acquisition means 71 acquires identification information of a subject (step S71). Next, the lesion information acquisition means 73 acquires the lesion information corresponding to the acquired identification information from the storage means 72 (step S72). The storage means 72 stores past examination data in which identification information of a subject is associated with a lesion image obtained by imaging a lesion, positional information of the lesion and lesion information including treatment performed for the lesion. Then, the display control means 74 display the acquired lesion information on the display device 75 (step S73).

According to the image display device 70 of the second example embodiment, at the time of an endoscopic examination, it is possible to advance the examination while considering the information about the treatment of the lesion found in the past endoscopic examinations.

A part or all of the above example embodiments may also be described as in the following supplementary notes, but are not limited to:

### (Supplementary note 1)

An image display device comprising:
a storage means configured to store past examination data in which identification information of a subject is associated with a lesion image obtained by imaging a lesion, positional information of the lesion and lesion information including treatment performed for the lesion;
an identification information acquisition means configured to acquire identification information of a subject;
a lesion information acquisition means configured to acquire the lesion information corresponding to the acquired identification information from the storage means; and
a display control means configured to display the lesion information on a display device.

### (Supplementary note 2)

The image display device according to Supplementary note 1, wherein, when the lesion information includes the lesion information for a plurality of lesions, the display control means displays the lesion information for a predetermined number of lesions selected in order of seriousness of diagnosis results.

### (Supplementary note 3)

The image display device according to Supplementary note 1, wherein, when the lesion information includes the lesion information for a plurality of lesions, the display control means simultaneously displays a plurality of lesion information related to the lesions belonging to a same part of an organ to be examined.

### (Supplementary note 4)

The image display device according to Supplementary note 3, further comprising an input means configured to receive a selection of one of a plurality of parts of the organ to be examined,
wherein the display control means displays the lesion information related to the lesion belonging to the selected part.

### (Supplementary note 5)

The image display device according to Supplementary note 1, further comprising an image acquisition means configured to acquire an endoscopic image during examination,
wherein the display control means displays the endoscopic image during the examination and the lesion information.

### (Supplementary note 6)

The image display device according to Supplementary note 5, wherein the display control means displays the lesion information related to the lesion belonging to a part, among a plurality of parts of an organ to be examined, which is same as the part indicated by the endoscopic image during the examination.

### (Supplementary note 7)

The image display device according to any one of Supplementary notes 1 to 6, wherein the positional information of the lesion indicates a part of an organ to be examined.

### (Supplementary note 8)

An image display method comprising:
acquiring identification information of a subject;
acquiring lesion information corresponding to the acquired identification information from a storage, the storage storing past examination data in which identification information of a subject is associated with a lesion image obtained by imaging a lesion, positional information of the lesion and lesion information including treatment performed for the lesion; and
displaying the lesion information on a display device.

### (Supplementary note 9)

A recording medium recording a program, the program causing a computer to execute processing of:
acquiring identification information of a subject;
acquiring lesion information corresponding to the acquired identification information from a storage, the storage storing past examination data in which identification information of a subject is associated with a lesion image obtained by imaging a lesion, positional information of the lesion and lesion information including treatment performed for the lesion; and
displaying the lesion information on a display device.

While the present disclosure has been described with reference to the example embodiments and examples, the present disclosure is not limited to the above example embodiments and examples. Various changes which can be understood by those skilled in the art within the scope of the present disclosure can be made in the configuration and details of the present disclosure.

### DESCRIPTION OF SYMBOLS

1 Image processing device
2 Display device
3 Endoscope
11 Processor
12 Memory
17 Database (DB)
21 Position detection unit
22 Examination data generation unit
23 Patient ID acquisition unit
24 Past data acquisition unit
25 Display data generation unit
100 Endoscopic examination system

## Claims

1. An image display device comprising:
a storage means configured to store past examination data in which identification information of a subject is associated with a lesion image obtained by imaging a lesion, positional information of the lesion and lesion information including treatment performed for the lesion;
an identification information acquisition means configured to acquire identification information of a subject;
a lesion information acquisition means configured to acquire the lesion information corresponding to the acquired identification information from the storage means; and
a display control means configured to display the lesion information on a display device.

2. The image display device according to claim 1, wherein, when the lesion information includes the lesion information for a plurality of lesions, the display control means displays the lesion information for a predetermined number of lesions selected in order of seriousness of diagnosis results.

3. The image display device according to claim 1, wherein, when the lesion information includes the lesion information for a plurality of lesions, the display control means simultaneously displays a plurality of lesion information related to the lesions belonging to a same part of an organ to be examined.

4. The image display device according to claim 3, further comprising an input means configured to receive a selection of one of a plurality of parts of the organ to be examined,
wherein the display control means displays the lesion information related to the lesion belonging to the selected part.

5. The image display device according to claim 1, further comprising an image acquisition means configured to acquire an endoscopic image during examination,
wherein the display control means displays the endoscopic image during the examination and the lesion information.

6. The image display device according to claim 5, wherein the display control means displays the lesion information related to the lesion belonging to a part, among a plurality of parts of an organ to be examined, which is same as the part indicated by the endoscopic image during the examination.

7. The image display device according to any one of claims 1 to 6, wherein the positional information of the lesion indicates a part of an organ to be examined.

8. An image display method comprising:
acquiring identification information of a subject;
acquiring lesion information corresponding to the acquired identification information from a storage, the storage storing past examination data in which identification information of a subject is associated with a lesion image obtained by imaging a lesion, positional information of the lesion and lesion information including treatment performed for the lesion; and
displaying the lesion information on a display device.

9. A recording medium recording a program, the program causing a computer to execute processing of:
acquiring identification information of a subject;
acquiring lesion information corresponding to the acquired identification information from a storage, the storage storing past examination data in which identification information of a subject is associated with a lesion image obtained by imaging a lesion, positional information of the lesion and lesion information including treatment performed for the lesion; and
displaying the lesion information on a display device.
